Europäisches Patentamt

European Patent Office

Office européen des brevets

(19)

(11) EP 1 078 993 A1

(12) DEMANDE DE BREVET EUROPEEN

(43) Date de publication:
28.02.2001 Bulletin 2001/09

(51) Int Cl.⁷: C12Q 1/68

(21) Numéro de dépôt: 99410103.8

(22) Date de dépôt: 24.08.1999

(84) Etats contractants désignés:
AT BE CH CY DE DK ES FI FR GB GR IE IT LI LU
MC NL PT SE
Etats d'extension désignés:
AL LT LV MK RO SI

(71) Demandeur: BIOMERIEUX
69280 Marcy-L'Etoile (FR)

(72) Inventeurs:
• Vachon, René
69100, Villeurbanne (FR)
• Lacroix, Bruno
69230, Saint-Genis Laval (FR)

(74) Mandataire: de Beaumont, Michel
1, rue Champollion
38000 Grenoble (FR)

(54) **Procédé d'analyse de mesures effectuées sur une biopuce**

(57) L'invention concerne un procédé d'analyse de mesures obtenues sur une biopuce comprenant une série de sondes de référence dont chacune est destinée à provoquer une hybridation avec une séquence spécifique d'un acide nucléique de référence, la séquence spécifique associée à une sonde étant décalée par rapport à la séquence spécifique associée à la sonde précédente d'une position correspondant à une ou plusieurs bases, et comprenant une série de sondes alternatives respectives dont chacune contient une mutation par rapport à sa sonde de référence. Ce procédé comprend les étapes suivantes pour identifier un acide nucléique cible :

mesurer les taux d'hybridation de séquences cible sur les sondes de référence et alternatives ;
déterminer les écarts entre les taux mesurés et des distributions préétablies respectives, représentant statistiquement où devraient se trouver les taux mesurés si la séquence cible était identique à la séquence de référence ; et
analyser les écarts pour des sondes de référence voisines et au moins une sonde alternative correspondante.

**Description**

La présente invention concerne les dispositifs d'analyse, appelés "biopuces", servant à identifier des analytes. L'invention concerne plus particulièrement un procédé d'analyse des mesures effectuées sur une telle biopuce.

Ce procédé d'analyse concerne la détection et/ou la quantification d'acides nucléiques à partir d'un prélèvement biologique quelconque. Avantageusement, le dispositif d'analyse permet l'identification d'acides nucléiques comme l'ADN simple ou double brin, les ARN messagers, ribosomaux ou de transfert obtenus directement à partir de l'échantillon biologique ou à partir d'une technique d'amplification enzymatique (voir par exemple l'article de E. Winn-Deen, Journal of Clinical Essay, vol 19, p. 21-26, (1996) pour les techniques d'amplification enzymatique).

Le procédé d'analyse est applicable au séquençage, à l'établissement de profils d'expression des ARN messagers ou au criblage de mutations, au diagnostic de maladies infectieuses ou génétiques, à l'identification de microorganismes ou à la détermination de mécanismes de résistance de ces microorganismes à des drogues.

Par biopuce, on entend tout support solide sur lequel sont fixés des ligands. La fixation des ligands peut être réalisée de différentes manières et notamment par adsorption ou covalence comme par exemple la synthèse in situ par des techniques de photolithographie ou par un système piézo-électrique, par dépôt capillaire de ligands préformés. A titre d'illustration, des exemples de ces biopuces sont donnés dans les publications de G. Ramsay, Nature Biotechnology, 16, p. 40-44, 1998 ; F. Ginot, Human Mutation, 10, p. 1-10, 1997 ; J. Cheng et al, Molecular diagnosis, 1(3), p. 183-200, 1996 ; T. Livache et al, Nucleic Acids Research, 22(15), p. 2915-2921, 1994 ; J. Cheng et al, Nature Biotechnology, 16, p. 541-546, 1998 ; ou dans les brevets US 4981783 (Augenlicht), 5700637 (Southern), 5445934 (Fodor), 5744305 (Fodor), 5807522 (Brown).

Les ligands fixés sur la biopuce sont des acides nucléiques et préférentiellement des oligonucléotides et sont fixés sur la biopuce par couplage covalent. Au moins 400 séquences différentes d'oligonucléotides et préférentiellement au moins 1000 sont fixées par cm$^2$ du support solide de la biopuce.

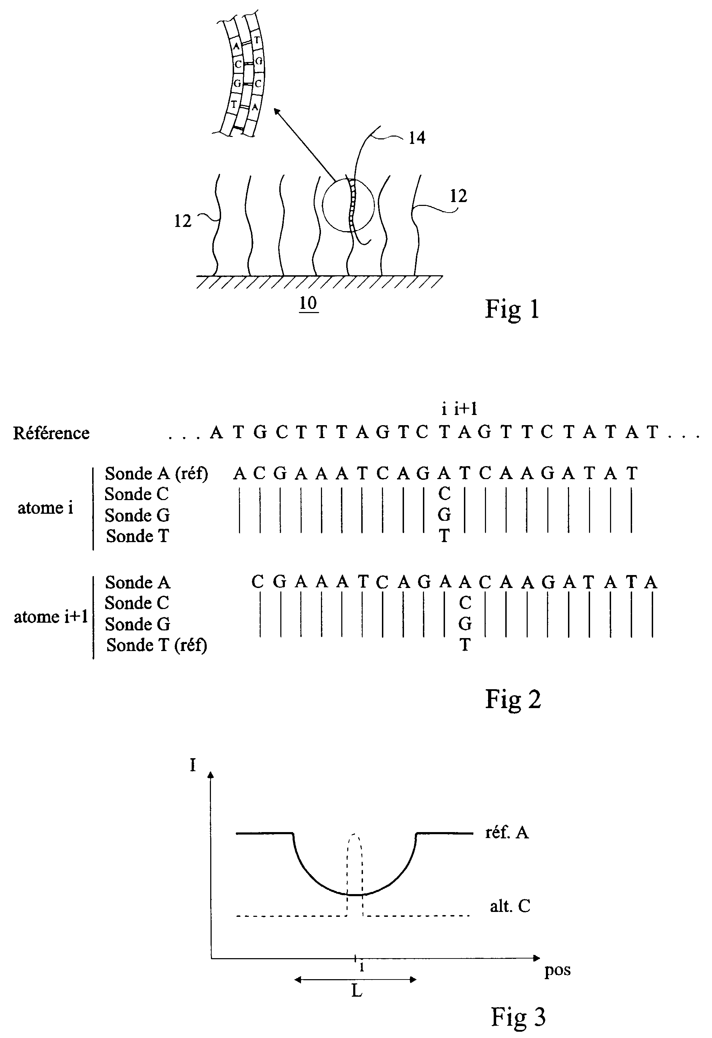

La figure 1 est une vue en coupe schématique et partielle d'une biopuce. Sur un support 10, on fixe des oligonucléotides 12 correspondant à une partie de séquence d'un acide nucléique à identifier. Chaque oligonucléotide 12 est en pratique une chaîne de 5 à 150 composants d'ADN élémentaires appelés nucléotides et caractérisés par la base (A, C, G, ou T) fixée sur le sucre du nucléotide, alors que les acides nucléiques à identifier peuvent comporter jusqu'à une dizaine de milliers de nucléotides.

Chaque oligonucléotide est susceptible d'entraîner une réaction d'hybridation avec l'acide nucléique comprenant une séquence complémentaire à celle de l'oligonucléotide, sachant que les bases A et C sont respectivement complémentaires des bases T et G. Une telle réaction est illustrée à la figure 1 où un acide nucléique 14 se fixe par une partie sur un oligonucléotide 12.

Une biopuce comporte généralement plusieurs cellules dont chacune regroupe plusieurs millions d'oligonucléotides identiques. Les oligonucléotides d'une cellule sont appelés "sonde".

Ainsi, en choisissant convenablement les sondes présentes sur une biopuce, on parvient à déterminer les acides nucléiques mis en présence de la biopuce en observant les sondes sur lesquelles se fixent ces acides nucléiques par hybridation.

Diverses solutions existent pour déterminer les taux d'hybridation des acides nucléiques sur les sondes. Une solution classique consiste à fixer sur les acides nucléiques à identifier des marqueurs fluorescents. On mesure alors l'intensité de fluorescence des diverses cellules de la puce, qui correspond directement au taux de concentration. Le procédé de la présente invention s'applique à d'autres méthodes de détection (voir par exemple DNA probes, G.H. Keller et M.M Manak, 2$^{nd}$ edition, Stockton Press, 1993, section 6).

Un problème résulte du fait que des acides nucléiques pourront se fixer sur des sondes même si les séquences ne correspondent pas exactement. Dans ce cas, l'hybridation entre l'acide nucléique et la sonde est plus faible que dans le cas d'une correspondance parfaite et les acides nucléiques ainsi fixés ont tendance à se détacher plus facilement lors d'un rinçage de la biopuce. Il en résulte que l'intensité relevée est alors plus faible que dans le cas d'un appariement parfait.

La figure 2 illustre une méthode classique de conception de biopuce visant à résoudre ce problème. Pour un acide nucléique comportant une séquence de référence de N bases, on réalise ce que l'on appelle un pavé 4L, également appelé "tile 4L", qui comporte près de N groupes appelés atomes, comportant chacun quatre sondes de longueur L (L ≈ 20). Chaque atome correspond à une position dans la séquence de référence (on a représenté les atomes pour les positions i et i+1 à la figure 2), et comporte une sonde dite "de référence" et trois autres sondes dites "alternatives" (il existe d'autres types de pavé, qui diffèrent de celui-ci par le nombre et la nature des sondes alternatives).

Dans un atome i, la sonde de référence correspond exactement à une partie de la séquence de référence couvrant la position i. Les trois autres sondes correspondent à la même partie de la séquence de référence, sauf qu'on a substitué en position i chacune des trois bases non complémentaires à celle de la séquence de référence à cette même position (cette substitution est de préférence effectuée dans le tiers central des sondes). On désigne alors les sondes alternatives par la base A, C, G ou T se trouvant à la position i.

Ainsi, dans l'exemple de la figure 2, la sonde A de l'atome i est la sonde de référence, puisqu'on trouve la base T à la position i de la séquence de référence. La sonde T est la sonde de référence de l'atome i+1, puisqu'on trouve la base A à la position i+1 de la séquence de référence, etc.

Avec cette méthode, la séquence de référence pour laquelle on constitue un pavé correspond le plus souvent à une séquence dite consensus ou sauvage pour une espèce ou un mécanisme de résistance à étudier. Alors, si les acides nucléiques effectivement mis en présence de la biopuce ont subi une mutation (substitution, insertion ou délétion), c'est-à-dire si l'espèce présente une séquence différente de la séquence de référence, la méthode permettra de déceler cette différence.

La figure 3 illustre la détection d'une substitution dans son principe. On suppose que l'acide nucléique étudié, appelé acide nucléique cible, présente à la position i une substitution de la base T par la base G par rapport à l'acide nucléique de référence. Jusqu'à la position i-L/2, l'intensité mesurée sur les sondes de référence correspondantes est normale. A partir de la position i-L/2, l'intensité s'abaisse pour atteindre une valeur minimale à la position i et remonte pour retrouver une valeur normale à la position i+L/2. Cette courbe d'intensité s'explique par le fait qu'un acide nucléique cible se fixe à la sonde de façon d'autant plus fragile que la position où il y a un désappariement entre une base de l'acide nucléique cible et une base de la sonde est proche du centre de la sonde.

Avec cette seule mesure d'intensité, on peut seulement déduire qu'il y a une substitution, sans toutefois pouvoir déterminer avec précision la position de la substitution ni la base qui a été substituée. Par contre, une analyse des intensités des sondes alternatives le permettra. En effet, avec le présent exemple, pour la sonde alternative C, on mesure une faible intensité pour toutes les positions sauf la position i où l'intensité présente un pic. En effet, pour toutes les positions sauf la position i, la sonde C présente une base désappariée en son centre.

Toutefois, en pratique, les courbes d'intensité relevées ne sont pas aussi nettes. En effet, la force de fixation des acides nucléiques sur les sondes, même en cas d'appariement parfait, dépend des conditions de mesure. En particulier, lorsque l'on met en présence les acides nucléiques à étudier et la biopuce, les conditions de réaction (température, composition du tampon d'hybridation...) ont une forte influence sur la force de fixation et devraient être adaptées à chaque sonde. Ceci n'étant pas possible, on effectue une adaptation moyenne de ces différentes conditions de réaction. Il en résulte que les mesures d'intensité varient fortement d'une sonde à l'autre.

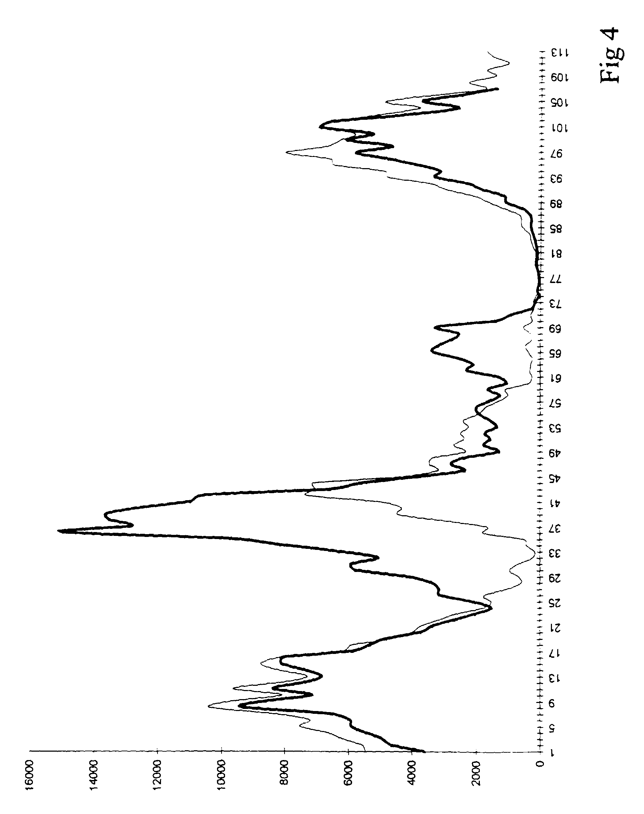

La figure 4 représente en gras un exemple de courbe d'intensité obtenue lorsque les acides nucléiques à identifier correspondent strictement aux sondes de référence. On a représenté en trait fin la courbe d'intensité obtenue pour un acide nucléique présentant une mutation par substitution aux positions 33 et 64.

Un algorithme classique pour détecter les substitutions dans l'acide nucléique étudié est l'algorithme des quotients. Cet algorithme déclare que la base présente à la position i est celle correspondant à la sonde pour laquelle on a relevé l'intensité maximale, à condition que le rapport entre cette intensité et l'intensité relevée sur toute autre sonde soit supérieur à un seuil prédéterminé, généralement 1,2. Si ce n'est pas le cas, on déclare qu'il y a une ambiguïté entre les deux bases correspondantes.

Cet algorithme est relativement efficace pour détecter toutes les substitutions. Néanmoins, notamment lorsque les intensités sont faibles (par exemple entre les positions 73 et 85 de la figure 4), il identifie de nombreuses fausses substitutions et trouve de nombreuses ambiguïtés. De plus, il ne permet pas d'identifier des mutations se caractérisant par une délétion ou une insertion d'une séquence dans la séquence de référence.

Un objet de la présente invention est de prévoir un procédé d'analyse de mesures effectuées sur une biopuce qui permette d'améliorer la qualité des résultats, notamment réduire les fausses déclarations de substitution et détecter des délétions ou insertions.

Pour atteindre cet objet, la présente invention prévoit un procédé d'analyse de mesures obtenues sur une biopuce comprenant une série de sondes de référence dont chacune est destinée à provoquer une hybridation avec une partie spécifique d'un acide nucléique de référence, la partie spécifique associée à une sonde étant décalée par rapport à la partie spécifique associée à la sonde précédente d'une position correspondant à une ou plusieurs bases, et comprenant une série de sondes alternatives respectives dont chacune contient une mutation par rapport à sa sonde de référence. Ce procédé comprend les étapes suivantes pour identifier un acide nucléique cible :

mesurer les taux d'hybridation de séquences cible sur les sondes de référence et alternatives ;
déterminer les écarts entre les taux mesurés et des distributions préétablies respectives, représentant statistiquement où devraient se trouver les taux mesurés si la séquence cible était identique à la séquence de référence ; et
analyser les écarts pour des sondes de référence voisines et au moins une sonde alternative correspondante.

Selon un mode de réalisation de la présente invention, ce procédé utilise un pavé 4L, ce pavé comportant une sonde de référence associée à trois sondes alternatives de sorte que les quatre sondes aient à une position les quatre bases possibles A, C, G et T.

Selon un mode de réalisation de la présente invention, l'analyse des écarts consiste à calculer, pour une sonde de référence courante, un indicateur qui est une combinaison linéaire des écarts relevés pour des sondes prises dans un voisinage de la sonde de référence courante, et à comparer cet indicateur à un premier seuil pour déterminer si la séquence cible contient la partie spécifique de la sonde de référence courante.

Selon un mode de réalisation de la présente invention, ce procédé comprend l'étape consistant à déclarer que la séquence cible contient la partie spécifique d'une sonde alternative si l'indicateur est supérieur au premier seuil et si l'écart mesuré pour cette sonde alternative est supérieur à un deuxième seuil.

Selon un mode de réalisation de la présente invention, ce procédé comprend les étapes consistant à, si l'écart mesuré pour plusieurs sondes alternatives respectives est supérieur au deuxième seuil, établir les quotients des taux mesurés pour les sondes alternatives respectives et du taux mesuré pour la sonde de référence ; et déclarer que la séquence cible contient la sonde alternative pour laquelle ledit quotient est le plus élevé.

Selon un mode de réalisation de la présente invention, l'indicateur n'intègre que les sondes de référence du voisinage et la sonde alternative respective pour laquelle le taux mesuré est le plus élevé.

Selon un mode de réalisation de la présente invention, ledit voisinage est excentré par rapport à la sonde de référence courante.

Selon un mode de réalisation de la présente invention, si aucune sonde alternative n'est déclarée, ce procédé comprend l'étape consistant à déclarer une délétion ou une insertion si un deuxième indicateur, qui est une combinaison linéaire des écarts relevés pour des sondes prises dans un voisinage de la sonde de référence courante, est supérieur à un troisième seuil et que l'écart mesuré pour toute sonde alternative respective est inférieur à un quatrième seuil.

Ces objets, caractéristiques et avantages, ainsi que d'autres de la présente invention seront exposés en détail dans la description suivante de modes de réalisation particuliers faite à titre non-limitatif en relation avec les figures jointes parmi lesquelles :

la figure 1, précédemment décrite, représente schématiquement et partiellement une vue en coupe d'une biopuce ;
la figure 2 illustre une configuration de biopuce dite à pavé 4L ;
la figure 3 représente schématiquement des mesures obtenues sur un pavé 4L dans le cas d'une substitution de base ;
la figure 4 représente des courbes d'intensité obtenues avec les sondes de référence d'un pavé pour une séquence de référence et une séquence mutée ;
la figure 5 est destinée à illustrer une étape préliminaire du procédé d'analyse selon l'invention pour un exemple de séquence de référence ;
la figure 6 illustre une étape principale du procédé selon l'invention permettant d'identifier une mutation par substitution ;
la figure 7 représente un tableau regroupant de manière simplifiée des mesures de distance selon l'invention pour des positions voisines d'une position de substitution ;
les figures 8A et 8B illustrent respectivement une délétion et une insertion détectables par le procédé de l'invention ; et
la figure 9 représente une courbe de distances selon l'invention pour la séquence mutée de la figure 4.

Le procédé d'analyse selon l'invention utilise une base de données statistique qui a été établie à partir de nombreuses mesures effectuées sur des biopuces en présence d'acides nucléiques de type sauvage. Les intensités relevées ont été classées dans des histogrammes de distribution, ces histogrammes étant enregistrés dans la base de données.

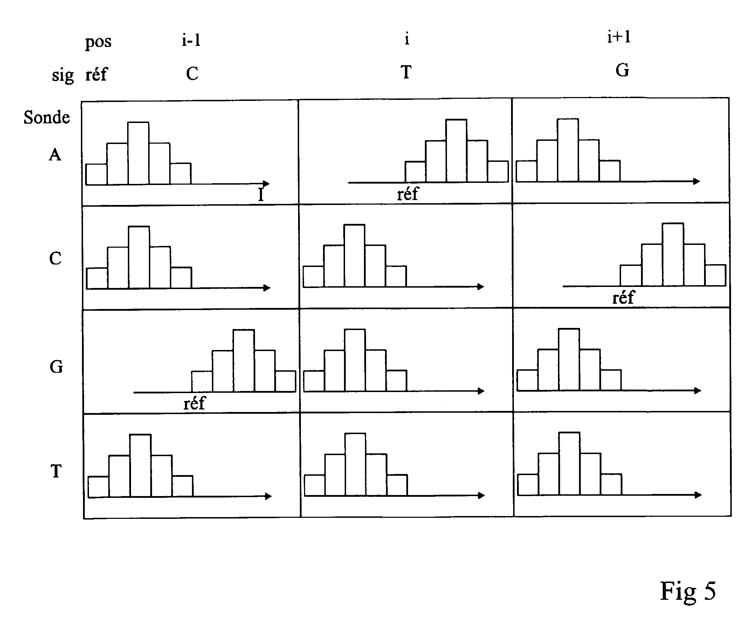

La figure 5 représente de manière schématique un extrait des histogrammes de la base de données pour des positions i-1, i et i+1 d'une séquence de référence ayant respectivement à ces positions les bases C, T et G. Ainsi, pour les positions i-1, i et i+1, les sondes de référence sont respectivement les sondes G, A et C. Pour ces sondes de référence, normalement, les intensités relevées sont nettement supérieures aux intensités relevées pour les sondes alternatives. Ainsi, les histogrammes pour les sondes de référence sont situés vers la droite tandis que les histogrammes pour les sondes alternatives sont situés vers la gauche, les intensités croissant de gauche à droite.

Il s'avère en pratique que les caractéristiques des biopuces varient notablement d'une puce à l'autre, même si les biopuces appartiennent à un même lot et sont utilisées dans les mêmes conditions. Il en résulte que les distributions statistiques, telles qu'exposées en relation avec la figure 5, seraient trop étalées pour être vraiment exploitables selon l'invention. En fait, les histogrammes de distribution de la base de données sont de préférence des histogrammes réalisés à partir de valeurs d'intensité normalisées, c'est-à-dire que chaque intensité mesurée est divisée par la moyenne de la distribution.

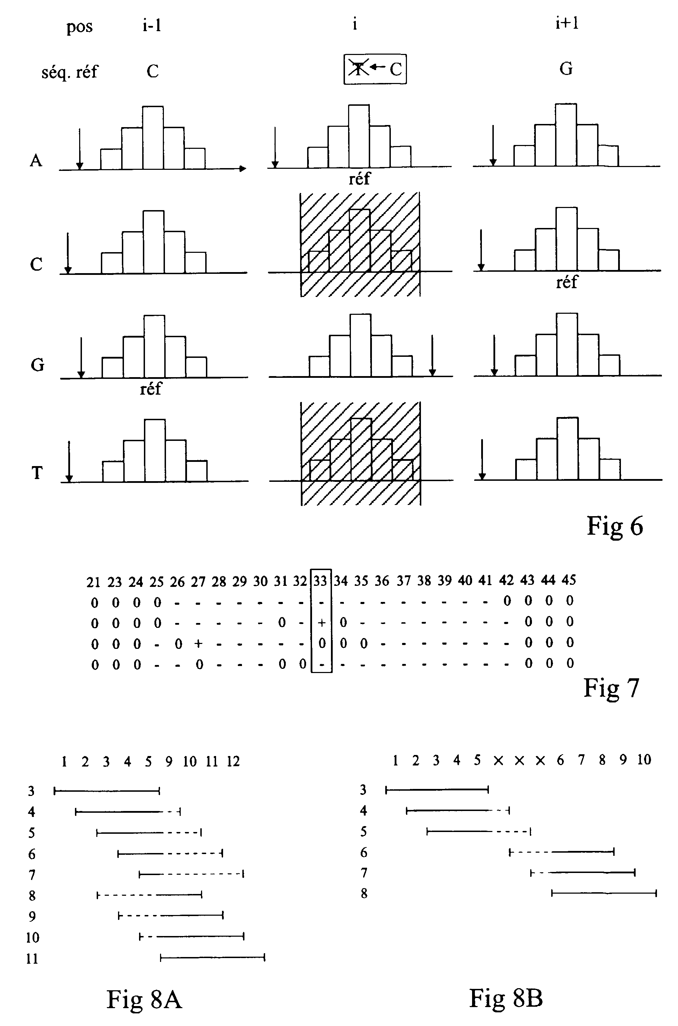

La figure 6 représente les histogrammes alors obtenus dans le même exemple que la figure 5. Tous les histogrammes sont centrés. On retiendra toutefois que ces histogrammes, même s'ils sont à la même position, correspondent à des échelles d'intensité différentes. En particulier, les histogrammes pour les sondes de référence correspondent à des intensités plutôt élevées tandis que les histogrammes pour les sondes alternatives correspondent à des intensités plutôt faibles.

Lors de l'analyse d'un acide nucléique selon l'invention, on extrait de la base de données les histogrammes associés au pavé utilisé pour effectuer les mesures. Les intensités sont relevées sur les sondes des différents atomes du pavé, et normalisées. Les intensités normalisées sont alors confrontées aux histogrammes correspondants.

Si la sonde de référence d'un atome est incluse dans l'acide nucléique analysé, les intensités normalisées mesurées tombent normalement dans les histogrammes.

La figure 6 illustre un cas où il y a une substitution de base à la position i. A titre d'exemple, la base T a été remplacée par la base C. Les intensités normalisées alors mesurées sont indiquées par des flèches ou des plages hachurées. Comme cela apparaît aux positions i-1 et i+1, les valeurs mesurées tombent en dessous des histogrammes. En effet, à ces positions, la sonde de référence comporte une base désappariée alors qu'elle devrait être parfaitement appariée, tandis que chacune des sondes alternatives comporte deux bases désappariées alors qu'elle devrait n'en comporter qu'une. Il en est de même de la position i-1 à la position i-L/2 et de la position i+1 à la position i+L/2, où L est le nombre de bases d'une sonde, ou la longueur de la sonde.

Par contre, ce qui est remarquable pour la position i, c'est que l'intensité relevée pour la sonde G tombe au-dessus de l'histogramme. En effet, la sonde G étant une sonde alternative, elle comporte normalement une base désappariée. Dans le présent cas, puisque la séquence cible comporte la base C au lieu de la base T à la position i, la sonde G s'avère être parfaitement appariée et l'intensité mesurée est plus élevée que la normale.

Les deux autres sondes alternatives (C et T) de position i comportent une base désappariée, ce qui correspond à la situation normale (sans substitution). Il en résulte que les valeurs mesurées correspondantes peuvent tomber dans les histogrammes, ce qui est indiqué par des plages hachurées.

L'intensité mesurée pour la sonde de référence (A) de position i est au-dessous de l'histogramme, comme pour les autres positions.

La figure 7 illustre une manière synthétique d'indiquer le résultat de la confrontation des valeurs mesurées aux histogrammes pour un acide nucléique présentant une mutation à une position donnée, la position 33 par exemple. Un résultat au-dessus d'un histogramme est représenté par le signe "+", un résultat en dessous par le signe "-", et un résultat à l'intérieur d'un histogramme par le signe "0".

Comme l'illustre cette figure 7, dans des conditions d'expérience réelles, les résultats ne sont pas binaires. En effet, on trouve des signes "0" ou "+" à des endroits où l'on devrait avoir le signe "-". Un biologiste qui examine les résultats présentés sous cette forme peut néanmoins déduire qu'il y a une substitution à la position 33, qui se trouve sensiblement au centre d'une plage où l'on trouve une majorité de signes "-". Il éliminera une possibilité de substitution à la position 27, où l'on trouve un signe "+", puisque cette position est trop proche du bord de la plage.

La présente invention permet également de déceler des parties omises ou des parties insérées dans la séquence étudiée par rapport à la séquence de référence.

La figure 8A est destinée à illustrer la détection d'une partie omise (ou délétion). Dans un but de simplification, on suppose que les sondes ont une longueur de cinq bases. Les sondes correspondant aux différentes positions sont représentées par des intervalles en face des parties correspondantes de la séquence cible.

Dans l'exemple de la figure 8A, les bases de positions 6, 7 et 8 sont omises. La sonde 3 correspond parfaitement aux positions 1 à 5. La sonde 4, destinée à correspondre aux positions 2 à 6 comporte une base désappariée, celle correspondant à la position 6. La sonde 5 comporte deux bases désappariées, celles correspondant aux positions 7 et 8. De même, chacune des sondes 6 à 10 comporte une ou plusieurs bases désappariées qui correspondent à des bases omises dans la séquence cible. Les sondes correspondent de nouveau parfaitement à partir de la sonde 11.

On s'aperçoit que, pour une partie omise de longueur D, on a L+D-1 sondes qui ne correspondent pas (L étant la longueur d'une sonde). Il en est ainsi, que les sondes soient de référence ou alternatives. Toutes les valeurs mesurées tombent donc au-dessous des histogrammes sur L+D-1 positions, ce qui permet, en théorie, de déterminer la longueur D de la partie omise.

La figure 8B illustre de la même manière que la figure 8A la détection d'une insertion dans la séquence cible. On suppose qu'il s'agit d'une insertion de trois bases entre les positions 5 et 6.

La sonde 3 correspond parfaitement aux positions 1 à 5. Les sondes 4 à 7 comportent une partie à cheval sur la partie ajoutée, d'où il résulte un désappariement de ces quatre sondes. La sonde 8, par contre, est en parfaite correspondance avec les positions 6 à 10.

On s'aperçoit que le nombre de sondes désappariées est égal à L-1, quelle que soit la longueur de l'insertion.

Les délétions ou les insertions peuvent être différenciées des substitutions par l'existence d'une valeur centrale mesurée au-dessus d'un histogramme pour une substitution (un signe "+" central dans le tableau de la figure 7) et l'absence d'une telle valeur pour les deux autres cas (pas de signe "+" central dans la figure 7). Par ailleurs, une délétion peut être différenciée d'une insertion par le fait que le nombre de sondes désappariées pour une délétion est strictement supérieur au nombre fixe (L-1) de sondes désappariées pour une insertion. Bien entendu, cette distinction est plus difficile à faire lorsque la délétion ne porte que sur une ou deux bases, compte tenu des variations dues aux conditions de mesure.

Les résultats d'analyse selon l'invention peuvent être présentés sous la forme de tableaux du type de la figure 7, à interpréter par le biologiste selon les différents critères que l'on vient de voir.

Toutefois, lorsque les valeurs mesurées sont de faible amplitude (par exemple, à la figure 4 entre les positions 73 et 85), les tableaux produits peuvent devenir plus difficiles à interpréter du fait de la présence d'un nombre élevé de signes erronés.

Pour améliorer la qualité des résultats, on utilisera de préférence, aussi bien pour créer les histogrammes de la base de données que pour confronter les valeurs mesurées à ces histogrammes, le logarithme des valeurs mesurées. Ceci a pour conséquence d'amplifier les écarts d'amplitude pour les basses amplitudes et de diminuer les écarts pour les amplitudes élevées.

Pour améliorer encore les résultats, on prévoit de calculer, pour chaque position, un indicateur spécifique. Si cet indicateur satisfait à des conditions spécifiques, on déclare qu'il y a une substitution à la position correspondante, sinon on déclare qu'il n'y a pas de substitution ou, selon les conditions, qu'il y a une ambiguïté. Cet indicateur est, par exemple, une combinaison linéaire de distances calculées pour des sondes au voisinage de la position associée.

Une distance correspondant à la position i, en utilisant la normalisation et le logarithme des valeurs mesurées, est exprimée par $d_{ij} = (O_{ij}-m_{ij})/s_{ij}$, où $j = 1$ pour la sonde de référence et $j = 2$, 3 ou 4 pour une sonde alternative, $O_{ij}$ est la valeur mesurée, $m_{ij}$ la moyenne de la distribution correspondante, et $S_{ij}$ l'écart-type de la distribution.

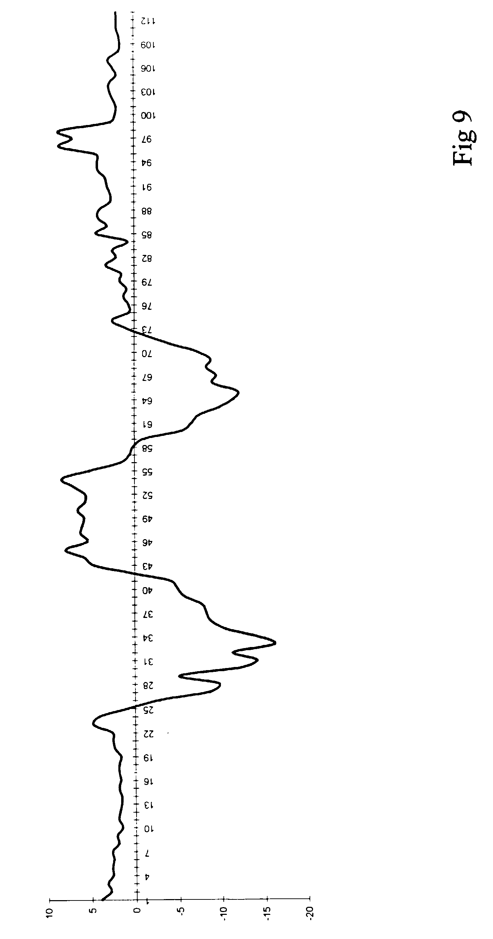

La figure 9 représente une courbe de variation des distances $d_{ij}$ ainsi définies pour la séquence mutée de la figure 4 (ayant subi une substitution aux positions 33 et 64).

On a essayé sur un échantillon plusieurs possibilités de combinaisons linéaires et de seuils pour les indicateurs, à savoir :

Jeu 1

SeuilDist = 2
SeuilSubst = 18
Indic = $-3d_{i-1,1}$ $-5d_{i1}$ $+5d_{iq}$ $-3d_{i+1,1}$

où q désigne la sonde alternative pour laquelle la valeur mesurée est au-dessus de la distribution.

Jeu 2

SeuilDist = 2
SeuilSubst = 10

$$\text{Indic} = -3d_{i-1,1} \; -2\sum_{j=2}^{4} d_{i-1,j} \; -5d_{i1} \; +5d_{iq} \; -3d_{i+1,1} \; -2\sum_{j=2}^{4} d_{i+1,j}$$

Jeu 3

SeuilDist = 2
SeuilSubst = 25
Indic = $-d_{i-3,1}$ $-2d_{i-2,1}$ $-3d_{i-1,1}$
        $+5d_{iq}$ $-5d_{i1}$
        $-3d_{i+1,1}$ $-2d_{i+2,1}$ $-d_{i+3,1}$

Jeu 4

SeuilDist = 2
SeuilSubst = 25
Indic = $-3d_{i-1,1}$ $-5d_{i1}$ $+5d_{iq}$ $-3d_{i+1,1}$ $-2d_{i+2,1}$ $-d_{i+3,1}$

Les jeux 1 et 3 ne tiennent compte que des sondes de référence et de la sonde alternative qui a fourni la mesure au-dessus de la distribution. Le jeu 1 intègre un voisinage symétrique minimal, tandis que le jeu 3 intègre un voisinage symétrique plus étendu.

Le jeu 2 est similaire au jeu 1, sauf qu'il intègre également toutes les sondes alternatives voisines.

Le jeu 4 intègre un voisinage excentré.

Ces indicateurs et seuils sont utilisés de la manière suivante. Si l'indicateur est supérieur à SeuilSubst et que la distance correspondant à l'une des sondes alternatives est supérieure à SeuilDist, on déclare qu'il y a une substitution par la base correspondant à ladite sonde alternative. Dans le cas contraire, on déclare qu'il n'y a pas de substitution.

Néanmoins, si ces conditions de substitution sont remplies pour deux sondes alternatives ou plus, on déclare qu'il y a une ambiguïté sur les bases correspondant auxdites sondes alternatives.

Ces jeux de combinaisons linéaires ont été testés sur 35 acides nucléiques cible de longueur 107, ce qui correspond à 35 x 107 = 3745 positions à étudier. Parmi ces positions, 25 correspondent à une substitution. Le tableau ci-dessous rassemble les résultats obtenus pour les différents jeux de combinaison linéaires :

| Jeu | Substitutions effectives détectées | | | | Substitutions détectées à tort | |
| --- | --- | --- | --- | --- | --- | --- |
| | Une seule base | | Doute entre deux bases | | | |
| | Nb | % | Nb | % | Nb | % |
| 1 | 23 | 92 | 2 | 8 | 11 | 0,29 |
| 2 | 23 | 92 | 2 | 8 | 29 | 0,78 |
| 3 | 23 | 92 | 2 | 8 | 10 | 0,27 |
| 4 | 23 | 92 | 2 | 8 | 9 | 0,24 |

On s'aperçoit que le jeu 4 semble fournir les meilleurs résultats, puisque c'est avec ce jeu que l'on détecte le moins de fausses substitutions. Par contre, on détecte de façon certaine seulement 23 substitutions parmi 25, mais on relève un doute entre deux bases substituées pour les deux substitutions restantes.

Le tableau ci-dessous fournit les résultats pour le même test en utilisant la méthode classique des quotients :

| Seuil | Substitutions effectives détectées | | | | Substitutions détectées à tort | | Doute sur une substitution alors qu'il n'y a pas de substitution | |
| --- | --- | --- | --- | --- | --- | --- | --- | --- |
| | Une seule base | | Doute entre deux bases | | | | | |
| | Nb | % | Nb | % | Nb | % | Nb | % |
| 1,2 | 25 | 100 | 0 | 0 | 71 | 1,9 | 37 | 0,99 |
| 1,0 | 25 | 100 | 0 | 0 | 95 | 2,55 | 0 | 0 |

On s'aperçoit que le nombre de substitutions détectées à tort est notablement plus élevé qu'avec le procédé selon l'invention. Par contre, cette méthode a permis de détecter toutes les vraies substitutions.

Cette méthode est globalement moins efficace que le procédé de l'invention, mais elle pourra être utilisée en complément du procédé de l'invention pour lever l'ambiguïté sur les substitutions non-détectées. Alors, on commence par employer le procédé de l'invention, utilisant les distributions de la base de données et les combinaisons linéaires susmentionnées, ce qui produira éventuellement un doute sur certaines substitutions. Pour ces substitutions douteuses, on utilise alors la méthode des quotients qui permettra de révéler que les substitutions douteuses sont effectivement des substitutions.

Pour détecter une délétion ou une insertion dans la séquence étudiée, on pourra également utiliser un indicateur qui est une combinaison linéaire de distances et utiliser deux seuils comme ci-dessus. On pourra par exemple utiliser :

SeuilDist = -2
SeuilDelIns = 190

$$\text{Indic} = -5d_{i1} -4,5d_{i-1,1} -3,5d_{i-2,1} -2,5d_{i-3,1} -4,5d_{i+1,1}$$
$$-3,5d_{i+2,1} -2,5d_{i+3,1} -1,5d_{i+4,1} -d_{i+5,1}$$
$$-\sum_{j=2}^{4} \left( 20d_{ij} +20d_{i-1,j} +20d_{i-2,j} +17,5d_{i-3,j} +12,5d_{i-4,j} \right.$$
$$\left. +2,5d_{i-5,j} +7,5d_{i+1,j} +5d_{i+2,j} +2,5d_{i+3,j} \right)$$

Si les conditions de substitution indiquées plus haut ne sont pas satisfaites, on pourra vérifier à l'aide de l'indicateur et des seuils ci-dessus s'il y a une délétion ou une insertion. Si l'indicateur ci-dessus est supérieur à SeuilDelIns et que la distance pour chacune des sondes alternatives est inférieure à SeuilDist, on déclare qu'il y a une délétion ou une insertion. La distinction entre une délétion et une insertion pourra alors être faite de la manière précédemment mentionnée par une observation d'un tableau du type de celui de la figure 7.

De nombreuses variantes et modifications de la présente invention apparaîtront à l'homme du métier. En particulier, il pourra utiliser l'une quelconque des méthodes disponibles pour déterminer le taux de concentration des acides nucléiques fixés sur les sondes. Dans la présente demande, on se réfère à titre d'exemple à la méthode du marquage fluorescent des acides nucléiques à étudier et l'analyse des intensités de fluorescence présentes sur la biopuce.

Les coefficients des combinaisons linéaires permettant de calculer les indicateurs sont purement indicatifs et l'homme du métier pourra les optimiser en fonction de son expérience.

Par ailleurs, dans la présente demande, on s'est référé à des pavés de type 4L comportant des atomes à quatre sondes. Il est envisageable, bien que cela soit moins avantageux, d'utiliser des atomes à deux sondes, à savoir une sonde de référence et une sonde correspondant à une mutation possible.

**Revendications**

**1.** Procédé d'analyse de mesures obtenues sur une biopuce comprenant une série de sondes de référence dont chacune est destinée à provoquer une hybridation avec une partie spécifique d'un acide nucléique de référence, la partie spécifique associée à une sonde étant décalée par rapport à la partie spécifique associée à la sonde précédente d'une position correspondant à une ou plusieurs bases, et comprenant une série de sondes alternatives respectives dont chacune contient une mutation par rapport à sa sonde de référence, caractérisé en ce qu'il comprend les étapes suivantes pour identifier un acide nucléique cible :

mesurer les taux d'hybridation de séquences cible sur les sondes de référence et alternatives ;
déterminer les écarts entre les taux mesurés et des distributions préétablies respectives, représentant statistiquement où devraient se trouver les taux mesurés si la séquence cible était identique à la séquence de référence ; et
analyser les écarts pour des sondes de référence voisines et au moins une sonde alternative correspondante.

**2.** Procédé d'analyse selon la revendication 1, caractérisé en ce qu'il utilise un pavé 4L, ce pavé comportant une sonde de référence associée à trois sondes alternatives de sorte que les quatre sondes aient à une position les quatre bases possibles A, C, G et T.

**3.** Procédé d'analyse selon la revendication 1 ou 2, caractérisé en ce que l'analyse des écarts consiste à calculer, pour une sonde de référence courante, un indicateur qui est une combinaison linéaire des écarts relevés pour des sondes prises dans un voisinage de la sonde de référence courante, et à comparer cet indicateur à un premier seuil pour déterminer si la séquence cible contient la partie spécifique de la sonde de référence courante.

**4.** Procédé d'analyse selon la revendication 3, caractérisé en ce qu'il comprend l'étape consistant à déclarer que la séquence cible contient la partie spécifique d'une sonde alternative si l'indicateur est supérieur au premier seuil et si l'écart mesuré pour cette sonde alternative est supérieur à un deuxième seuil.

**5.** Procédé d'analyse selon la revendication 4, caractérisé en ce qu'il comprend les étapes suivantes :

si l'écart mesuré pour plusieurs sondes alternatives respectives est supérieur au deuxième seuil, établir les quotients des taux mesurés pour les sondes alternatives respectives et du taux mesuré pour la sonde de référence ; et
déclarer que la séquence cible contient la sonde alternative pour laquelle ledit quotient est le plus élevé.

**6.** Procédé d'analyse selon la revendication 3, 4 ou 5, caractérisé en ce que l'indicateur n'intègre que les sondes de référence du voisinage et la sonde alternative respective pour laquelle le taux mesuré est le plus élevé.

**7.** Procédé d'analyse selon la revendication 3, caractérisé en ce que ledit voisinage est excentré par rapport à la sonde de référence courante.

**8.** Procédé d'analyse selon la revendication 4, caractérisé en ce que, si aucune sonde alternative n'est déclarée, il comprend l'étape consistant à déclarer une délétion ou une insertion si un deuxième indicateur, qui est une combinaison linéaire des écarts relevés pour des sondes prises dans un voisinage de la sonde de référence courante, est supérieur à un troisième seuil et que l'écart mesuré pour toute sonde alternative respective est inférieur à un quatrième seuil.

Fig 1

i i+1

Référence ... A T G C T T T A G T C T A G T T C T A T A T ...

Fig 2

Fig 3

Fig 4

Fig 5

pos    i-1        i        i+1

séq. réf    C      X←C      G

A

réf

C

réf

G

réf

T

**Fig 6**

| 21 | 23 | 24 | 25 | 26 | 27 | 28 | 29 | 30 | 31 | 32 | 33 | 34 | 35 | 36 | 37 | 38 | 39 | 40 | 41 | 42 | 43 | 44 | 45 |
|---|---|---|---|---|---|---|---|---|---|---|---|---|---|---|---|---|---|---|---|---|---|---|---|
| 0 | 0 | 0 | 0 | - | - | - | - | - | - | - | - | - | - | - | - | - | - | - | - | 0 | 0 | 0 | 0 |
| 0 | 0 | 0 | 0 | - | - | - | - | - | 0 | - | + | 0 | - | - | - | - | - | - | - | | 0 | 0 | 0 |
| 0 | 0 | 0 | - | 0 | + | - | - | - | - | - | 0 | 0 | 0 | - | - | - | - | - | - | - | - | 0 | 0 | 0 |
| 0 | 0 | 0 | - | - | 0 | - | - | - | 0 | 0 | - | - | - | - | - | - | - | - | - | - | 0 | 0 | 0 |

**Fig 7**

1 2 3 4 5 9 10 11 12

3
4
5
6
7
8
9
10
11

**Fig 8A**

1 2 3 4 5 × × × 6 7 8 9 10

3
4
5
6
7
8

**Fig 8B**

Fig 9

**Office européen
des brevets**

**RAPPORT DE RECHERCHE EUROPEENNE**

Numéro de la demande

EP 99 41 0103

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.7) |
|---|---|---|---|
| X | GUNDERSON ET AL.: "MUTATION DETECTION BY LIGATION TO COMPLETE N-MER DNA ARRAYS" GENOME RESEARCH, vol. 8, 1998, pages 1142-1153, XP002130857 * le document en entier * | 1-8 | C12Q1/68 |
| X | EP 0 717 113 A (AFFYMAX TECH NV) 19 juin 1996 (1996-06-19) * le document en entier * | 1-8 | |
| X | WO 97 29212 A (GINGERAS THOMAS A ;CHEE MARK S (US); STRYER LUBERT (US); AFFYMETRI) 14 août 1997 (1997-08-14) * le document en entier * | 1-8 | |
| X | EP 0 785 280 A (AFFYMETRIX INC) 23 juillet 1997 (1997-07-23) * le document en entier * | 1-8 | |
| X | WO 95 11995 A (AFFYMAX TECH NV ;FODOR STEPHEN P A (US); GINGERAS THOMAS R (US); L) 4 mai 1995 (1995-05-04) * le document en entier * | 1-8 | **DOMAINES TECHNIQUES RECHERCHES (Int.Cl.7)** C12Q |
| X | TROESCH ET AL.: "MYCOBACTERIUM SPECIES IDENTIFICATION AND RIFAMPICIN RESISTANCE TESTING WITH HIGH-DENSITY DNA PROBE ARRAYS" J.CLIN.MICROBIOLOGY, vol. 37, no. 1, janvier 1999 (1999-01), pages 49-55, XP002130858 * le document en entier * | 1-8 | |

-/--

Le présent rapport a été établi pour toutes les revendications

| Lieu de la recherche | Date d'achèvement de la recherche | Examinateur |
|---|---|---|
| LA HAYE | 18 février 2000 | Hagenmaier, S |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

........................................................................
& : membre de la même famille, document correspondant

EPO FORM 1503 03.82 (P04C02)

**Office européen**

**des brevets**

**RAPPORT DE RECHERCHE EUROPEENNE**

Numéro de la demande

EP 99 41 0103

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.7) |
|---|---|---|---|
| X | WANG D G ET AL: "Large-scale identification, mapping, and genotyping of single-nucleotide polymorphisms in the human genome" SCIENCE,US,AMERICAN ASSOCIATION FOR THE ADVANCEMENT OF SCIENCE,, vol. 280, 1 janvier 1998 (1998-01-01), pages 1077-1082, XP002089398 ISSN: 0036-8075 * le document en entier * ----- | 1-8 | |
| | | | **DOMAINES TECHNIQUES RECHERCHES (Int.Cl.7)** |

Le présent rapport a été établi pour toutes les revendications

| Lieu de la recherche | Date d'achèvement de la recherche | Examinateur |
|---|---|---|
| LA HAYE | 18 février 2000 | Hagenmaier, S |

CATEGORIE DES DOCUMENTS CITES

X : particulièrement pertinent à lui seul
Y : particulièrement pertinent en combinaison avec un autre document de la même catégorie
A : arrière–plan technologique
O : divulgation non–écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)

**ANNEXE AU RAPPORT DE RECHERCHE EUROPEENNE
RELATIF A LA DEMANDE DE BREVET EUROPEEN NO.**
EP 99 41 0103

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

18-02-2000

| Document brevet cité au rapport de recherche | | Date de publication | Membre(s) de la famille de brevet(s) | | Date de publication |
|---|---|---|---|---|---|
| EP 0717113 | A | 19-06-1996 | US | 5795716 A | 18-08-1998 |
| | | | US | 5974164 A | 26-10-1999 |
| WO 9729212 | A | 14-08-1997 | AU | 2189397 A | 28-08-1997 |
| | | | EP | 0937159 A | 25-08-1999 |
| EP 0785280 | A | 23-07-1997 | US | 5858659 A | 12-01-1999 |
| WO 9511995 | A | 04-05-1995 | AU | 8126694 A | 22-05-1995 |
| | | | EP | 0730663 A | 11-09-1996 |
| | | | JP | 9507121 T | 22-07-1997 |
| | | | US | 5861242 A | 19-01-1999 |
| | | | US | 5837832 A | 17-11-1998 |

EPO FORM P0460

Pour tout renseignement concernant cette annexe : voir Journal Officiel de l'Office européen des brevets, No.12/82